# EUROPEAN PATENT APPLICATION

(11) **EP 4 647 119 A1**
(43) Date of publication of application: **12.11.2025**
(21) Application number: 24738707.9
(22) Date of filing: 03.01.2024
(51) Int. Cl.: A61N 7/00, A61B 34/10, A61B 6/03, A61B 6/00, A61B 5/055, B33Y 50/00, B33Y 80/00

(54) **THERAPEUTIC ULTRASONICATION SYSTEM FOR OPENING BLOOD-BRAIN BARRIER AND ULTRASONIC WAVE CONTROL METHOD OF THERAPEUTIC ULTRASONICATION SYSTEM FOR OPENING BLOOD-BRAIN BARRIER**

(30) Priority: 03.01.2023 KR 20230000929; 14.12.2023 KR 20230181361
(71) Applicant: Neumous Inc., Cheongju-si, Chungcheongbuk-do 28160 (KR)
(72) Inventor: PARK, Juyoung, Seongnam-si Gyeonggi-do 13643 (KR); PARK, Chan Yuk, Seoul 03668 (KR)
(74) Representative: Schmitt-Nilson Schraud Waibel Wohlfrom Patentanwälte Partnerschaft mbB
(86) International application number: PCT/KR2024/000119
(87) International publication number: WO 2024/147637

(57) **Abstract**

The present invention relates to an ultrasonic wave control method of a therapeutic sonication system for opening a blood-brain barrier, the method comprising the steps of: setting output conditions for outputting ultrasonic energy to an acoustic cavitation-inducing substance administered to a subject; outputting ultrasonic energy corresponding to the output conditions to the subject; receiving an acoustic signal resulting from acoustic cavitation induced by the acoustic cavitation-inducing substance; analyzing the acoustic signal to determine whether preset control conditions have been satisfied; and, depending on whether the preset control conditions have been satisfied, outputting ultrasonic energy after adjusting at least one of a duty cycle, the number of stimulation pulses per a predetermined time unit, pulse repetition frequency per a predetermined time unit, and applied voltage, among the output conditions of ultrasonic energy, or stopping the outputting of ultrasonic energy.

## Description

### [TECHNICAL FIELD]

The present disclosure relates to a therapeutic ultrasonication system for blood-brain barrier opening and an ultrasonic wave control method of the therapeutic ultrasonication system.

### [BACKGROUND ART]

The present disclosure results from research conducted as part of the Drug Delivery Technology Development Project of the Ministry of Health and Welfare (Project No.: 1465040354, Task No.: HI23C0344000023, Research Project Name: Development of Brain Drug Delivery Technology Using Externally Attached Ultrasound and Drug Carriers, Project Managing Institution: Korea Health Industry Development Institute, Project Performing Institution: NUMUS Inc., Research Period: April 1, 2023 ~ December 31, 2027, and Contribution Rate: 50%), and the Startup Growth Technology Development (Materials, Parts, and Equipment Accounting) of the Ministry of SMEs and Startups (Project No.: 1425179650, Task No.: 00261874, Research Project Name: Development of Patient-Customized Blood-Brain Barrier Modulating Multi-Channel Focused Ultrasound Equipment, Project Managing Institution: Korea Technology and Information Promotion Agency for SMEs, Project Performing Institution: NUMUS Inc., Research Period: June 1, 2023 ~ May 31, 2026, and Contribution Rate: 50%). Meanwhile, there is no property interest of the Korean government in any aspect of the present disclosure.

The blood-brain barrier (BBB) refers to a physiological barrier which is present in a brain-blood vessel to separate and protect the brain and the central nervous system. The barrier separates neural cells and the blood in the brain.

Meanwhile, the phenomenon that a brain disease therapeutic agent fails to sufficiently penetrate into the brain due to the blood-brain barrier is an important issue in association with many neurological and medical challenges. That is, the blood-brain barrier separates the blood and the brain tissue to protect and safely maintain the brain and simultaneously makes the effective transfer of the brain disease therapeutic agent difficult.

Nowadays, various technologies for transferring a brain disease therapeutic agent through the blood-brain barrier are being developed and studied, but there are various limitations such as a limited or temporary effect, a safety issue, or difficulty in accurate targeting.

Accordingly, there is a need for a technology capable of efficiently and safely controlling the opening of the blood-brain barrier to overcome the above issues and to effectively transfer therapeutic agents for brain diseases into the brain.

### [DETAILED DESCRIPTION OF THE INVENTION]

### [TECHINICAL PROBLEM]

The technical problem to be solved by the present disclosure is to control an ultrasound irradiation parameter by analyzing, in real time, an acoustic signal generated by the motion (or translational/oscillatory motion) of microbubbles due to the interaction between microbubbles injected into a blood vessel and ultrasound stimulation irradiated to a target brain for blood-brain barrier opening.

In this way, it is possible to control the degree of opening of the blood-brain barrier in real time, thereby minimizing damage of perivascular tissue and safely opening the blood-brain barrier.

### [TECHNICAL SOLUTION]

According to an embodiment of the present disclosure, an ultrasonic wave control method of a therapeutic ultrasonication system for blood-brain barrier opening may include setting, by at least one processor, an output condition for outputting ultrasound energy to an acoustic cavitation generation material injected into an object, outputting, by the at least one processor, ultrasound energy corresponding to the output condition to the object, receiving, by the at least one processor, an acoustic signal due to an acoustic cavitation phenomenon of the acoustic cavitation generation material, analyzing, by the at least one processor, the acoustic signal to determine whether a preset control condition is satisfied, and stopping, by the at least one processor, the output of the ultrasound energy or adjusting at least one of output conditions to output the ultrasound energy, depending on whether the preset control condition is satisfied. The output conditions may include a duty cycle, the number of stimulus pulses per preset time, a pulse repetition frequency per preset time, and an applied voltage.

According to an embodiment of the present disclosure, setting an initial center frequency or an initial applied voltage among the output conditions and setting at least one of an initial duty cycle, the initial number of stimulus pulses per preset time, and an initial pulse repetition frequency per preset time among the output conditions may be included.

According to an embodiment of the present disclosure, outputting, to the object, the ultrasound energy having at least one of the set initial center frequency, the set initial duty cycle, the set initial number of stimulus pulses, and the set initial pulse repetition frequency or the ultrasound energy generated by applying the set initial applied voltage may be included, and receiving the acoustic signal from the acoustic cavitation generation material injected into the object may be included.

According to an embodiment of the present disclosure, performing a frequency analysis on the acoustic signal, determining whether a broadband noise is present in the frequency analysis, analyzing a value of sub-harmonics or ultra-harmonics in the frequency analysis, and analyzing the value of the sub-harmonics or the ultra-harmonics to determine whether to reach a preset first threshold value may be included.

According to an embodiment of the present disclosure, stopping the output of the ultrasound energy, when the broadband noise exists, stopping the output of the ultrasound energy, when the value of the sub-harmonics or the ultra-harmonics reaches the preset first threshold value, and analyzing a value of harmonics in the frequency analysis, when the broadband noise does not exist and when the value of the sub-harmonics or the ultra-harmonics does not reach the preset first threshold value may be included.

According to an embodiment of the present disclosure, determining whether the value of the harmonics reaches a preset second threshold value, and adjusting at least one of the duty cycle, the number of stimulus pulses per preset time, the pulse repetition frequency per preset time, and the applied voltage, when the value of the harmonics does not reach the preset second threshold value may be included.

According to an embodiment of the present disclosure, increasing at least one of the duty cycle, the number of stimulus pulses per preset time, the pulse repetition frequency per preset time, and the applied voltage such that an intensity of the ultrasound energy to be output is increased, when the value of the harmonics does not reach the preset second threshold value, and determining whether the sub-harmonics or the ultra-harmonics exist or whether the value of the sub-harmonics or the value of the ultra-harmonics reaches a preset third threshold value, when the value of the harmonics reaches the preset second threshold value may be included.

According to an embodiment of the present disclosure, increasing at least one or more of the duty cycle, the number of stimulus pulses per preset time, the pulse repetition frequency per preset time, and the applied voltage such that the intensity of the ultrasound energy to be output is increased, when the sub-harmonics or the ultra-harmonics do not exist or when the value of the sub-harmonics or the value of the ultra-harmonics does not reach the preset third threshold value, and determining whether a preset treatment time or a preset output time arrives, when the sub-harmonics or the ultra-harmonics exist or when the value of the sub-harmonics or the value of the ultra-harmonics reaches the preset third threshold value may be included.

According to an embodiment of the present disclosure, stopping the output of the ultrasound energy, when the preset treatment time arrives, and decreasing at least or more of the duty cycle, the number of stimulus pulses per preset time, the pulse repetition frequency per preset time, and the applied voltage such that the intensity of the ultrasound energy to be output is decreased, when the preset treatment time does not arrive may be included.

According to an embodiment of the present disclosure, stopping the output of the ultrasound energy, when the preset output time arrives, and decreasing at least one of the duty cycle, the number of stimulus pulses per preset time, the preset pulse repetition frequency, and the applied voltage such that the intensity of the ultrasound energy to be output is decreased, when the preset output time does not arrive may be included.

A non-transitory computer-readable recording medium may be provided which stores a program for executing the ultrasonic wave control method of the therapeutic ultrasonication system for blood-brain barrier opening according to an embodiment of the present disclosure.

According to an embodiment of the present disclosure, a therapeutic ultrasonication system for blood-brain barrier opening may include a signal generation condition setting unit that sets an output condition for outputting ultrasound energy to an acoustic cavitation generation material injected into an object, a signal generation unit, the ultrasound energy corresponding to the output condition being output to the object under control of the signal generation unit, a signal collection unit that receives an acoustic signal due to an acoustic cavitation phenomenon of the acoustic cavitation generation material, a frequency analysis unit that analyzes the acoustic signal to determine whether to satisfy a preset control condition, and a signal control unit that stops the output of the ultrasound energy or adjusts at least one of output conditions of the ultrasound energy, depending on whether the preset control condition is satisfied. The conditions may include a duty cycle, the number of stimulus pulses per preset time, a pulse repetition frequency per preset time, and an applied voltage.

### [ADVANTAGEOUS EFFECTS OF THE INVENTION]

According to the present disclosure, the therapeutic ultrasonication system for blood-brain barrier opening and a control method of the therapeutic ultrasonication system may control the output of the ultrasound energy based on the acoustic signal received from the microbubble near the blood-brain barrier, and thus, it may be possible to minimize damage of perivascular tissue of the blood-brain barrier and also to open the blood-brain barrier stably.

### [DESCRIPTION OF THE DRAWINGS]

FIG. 1 is a diagram illustrating a therapeutic ultrasonication system for blood-brain barrier opening, according to an embodiment of the present disclosure.
FIG. 2 is a flowchart illustrating an ultrasound control method (or closed-loop feedback) of a therapeutic ultrasonication system for blood-brain barrier opening, according to an embodiment of the present disclosure.
FIG. 3A is a diagram illustrating ultrasound energy according to a change in a duty cycle, according to an embodiment of the present disclosure.
FIG. 3B is a diagram illustrating ultrasound energy according to a change in an applied voltage, according to an embodiment of the present disclosure.
FIG. 3C is a diagram illustrating ultrasound energy according to a change in the number of stimulus pulses per preset time, according to an embodiment of the present disclosure.
FIG. 3D is a diagram illustrating ultrasound energy according to a change in a pulse repetition frequency per preset time, according to an embodiment of the present disclosure.
FIG. 4 is a diagram illustrating an analysis result of an acoustic signal according to a change in the number of cycles, according to an embodiment of the present disclosure.
FIG. 5 is a diagram illustrating an analysis result of an acoustic signal according to closed-loop feedback, according to an embodiment of the present disclosure.

### [BEST MODE]

Hereinafter, various embodiments of the present disclosure will be described in detail with reference to the accompanying drawings such that one skilled in the art to which the present disclosure pertains readily carries out the present disclosure. The present disclosure may be implemented in various different forms and is not limited to embodiments described herein.

Components or elements not associated with the detailed description may be omitted to describe the present disclosure clearly, and the same reference numerals/signs refer to the same or similar components throughout the specification. Therefore, the reference numerals/signs described above may be used in other drawings as well.

Moreover, because the size and thickness of each of the components illustrated in the drawings are shown arbitrarily for convenience of description, and the present disclosure is not necessarily limited thereto. In drawings, the thickness may be exaggerated to clearly express various layers and regions.

Besides, the expression "the same as" in the description may mean "substantially the same as". In other words, "the same" may mean "the same" to such an extent as a person with ordinary knowledge is capable of understanding. Other expressions may also be expressions in "substantially" is omitted.

Furthermore, when any portion "includes/comprises" a component in the detailed description, it will be understood that it may further include another component, without excluding other components unless specifically stated otherwise. The term "~ part" or "~ unit" used herein is used as a unit for processing at least one function or operation, and may mean, for example, software, an FPGA, or a hardware component. A function provided by "~ part" or "~ unit" may be distributed and performed by a plurality of components, or may be integrated with any other additional component(s). In the specification, the term "~ part" or "~ unit" is not necessarily limited to software or hardware, and may be configured to reside on an addressable storage medium or to reproduce one or more processors. Hereinafter, an embodiment of the present disclosure will be described in detail with reference to the accompanying drawings.

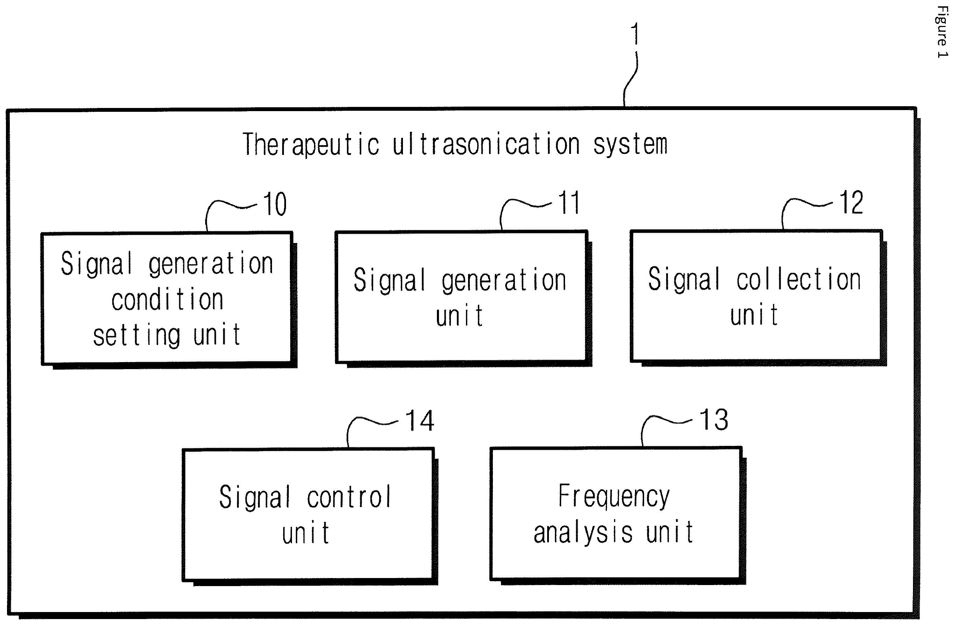

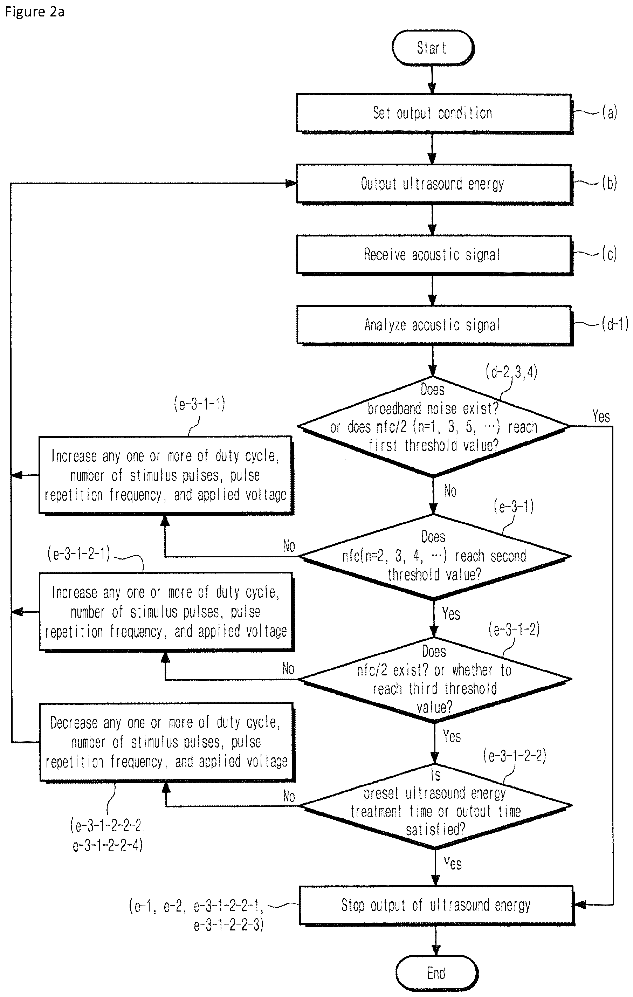

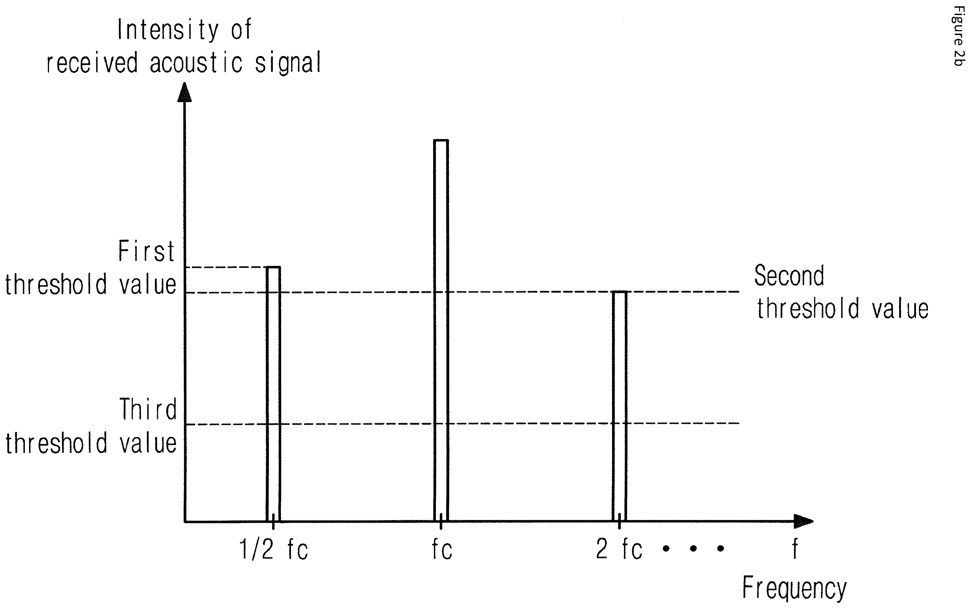

FIG. 1 is a diagram illustrating a therapeutic ultrasonication system for blood-brain barrier opening, according to an embodiment of the present disclosure. FIG. 2 is a flowchart illustrating an ultrasound control method (or closed-loop feedback) of a therapeutic ultrasonication system for blood-brain barrier opening, according to an embodiment of the present disclosure.

Below, the description will be given with reference to FIGS. 1 and 2A together.

A therapeutic ultrasonication system 1 for blood-brain barrier opening according to an embodiment of the present disclosure may include a signal generation condition setting unit 10, a signal generation unit 11, a signal collection unit 12, a frequency analysis unit 13, and a signal control unit 14.

However, it is obvious that fewer or more components than the components illustrated in FIG. 1 may be included to drive the therapeutic ultrasonication system 1 for blood-brain barrier opening.

For example, the therapeutic ultrasonication system 1 for blood-brain barrier opening may further include a power supply unit (not illustrated) which is connected to the therapeutic ultrasonication system 1 for blood-brain barrier opening to supply a power, an output or treatment time control unit (not illustrated) for setting or controlling an output time or treatment time of ultrasound energy, a piezoelectric element (not illustrated) which receives an electrical signal and converts and outputs the electrical signal into ultrasound energy or which receives ultrasound energy and converts the ultrasound energy into an electrical signal, a resonant circuit (not illustrated) which allows ultrasound energy to have a relevant resonance frequency such that ultrasound energy with an arbitrary resonance (or center) frequency set by the user is output, and a display unit (not illustrated) which is capable of visually checking a frequency analysis result.

Each of the signal generation condition setting unit 10, the signal generation unit 11, the signal collection unit 12, the frequency analysis unit 13, and the signal control unit 14 may be driven by at least one processor.

At step (a), the signal generation condition setting unit 10 may set an output condition for outputting ultrasound energy to an acoustic cavitation generation material injected into an object.

In detail, the acoustic cavitation generation material (e.g., microbubbles) may be in advance injected into the object.

The at least one processor which drives the signal generation condition setting unit 10 may set the output condition of the ultrasound energy which is output by the piezoelectric element.

For example, the output condition may be at least one of a duty cycle of ultrasound energy, the number of stimulus pulses per preset time, a pulse repetition frequency (PRF) per preset time, and an applied voltage to be provided to the piezoelectric element.

At step (a-1), the at least one processor may set an initial center frequency of ultrasound energy or an initial applied voltage to be provided to the piezoelectric element. At step (a-2), the at least one processor may set at least one of an initial duty cycle of ultrasound energy, the preset number of initial stimulus pulses, and an initial pulse repetition frequency per preset time.

At step (b), under control of the signal generation unit 11, ultrasound energy satisfying a relevant output condition may be output to an object.

In detail, the at least one processor which drives the signal generation unit 11 may provide a relevant electrical signal to the piezoelectric element such that ultrasound energy having at least one of the initial center frequency, the initial duty cycle, the number of initial stimulus pulses, and the initial pulse repetition frequency, which are thus set, is output to the acoustic cavitation generation material injected into the object.

Alternatively, the at least one processor which drives the signal generation unit 11 may provide an electrical signal having the set initial applied voltage to the piezoelectric element; in this case, under control of the at least one processor, the piezoelectric element may output relevant ultrasound energy to the acoustic cavitation generation material.

According to the above description, the microbubbles which constitute the acoustic cavitation generation material may be near the blood-brain barrier (BBB) in the object, and under control of the signal generation unit 11, the piezoelectric element may output ultrasound energy to the microbubbles (or a brain target) near the blood-brain barrier.

At step (c), the signal collection unit 12 may receive an acoustic signal due to the acoustic cavitation of the acoustic cavitation generation material.

In detail, the acoustic cavitation generation material which is supplied with the ultrasound energy may oscillate by the resonance phenomenon, and thus, the acoustic signal may be generated. In this case, an ultrasonic piezoelectric element for reception may detect a relevant acoustic signal, that is, the received acoustic signal and may convert the acoustic signal into an electrical signal.

That is, the piezoelectric element (or piezoelectric element for output) which outputs the ultrasound energy may be the same as or different from the piezoelectric element (or piezoelectric element for reception) which receives the ultrasound energy.

The at least one processor which drives the signal collection unit 12 may perform the conversion to the electrical signal.

At step (d), the frequency analysis unit 13 may analyze the received acoustic signal to determine whether the received acoustic signal satisfies a preset control condition.

At step (d), the frequency analysis unit 13 may analyze the received acoustic signal to determine whether the received acoustic signal satisfies the preset control condition even though the received acoustic signal satisfies at least one of step (d-2), step (d-3), step (d-4), step (e-3-1), step (e-3-1-2), and step (e-3-1-2-2) to be described below.

In detail, at step (d-1), the at least one processor which constitutes the frequency analysis unit 13 may analyze the received acoustic signal (or may analyze the frequency of the received acoustic signal). In this case, the Fourier transform or the like may be used, but the present disclosure is not limited thereto. That is, various techniques capable of analyzing the acoustic signal may be used.

At step (d-2), the at least one processor may determine whether a broadband noise (or a wideband noise) is present in the frequency analysis.

The broadband noise means a noise which is generated in a very wide frequency range. When the broadband noise exists, it may be determined that the phenomenon that the microbubbles near the blood-brain barrier are destroyed by ultrasound irradiation occurs.

That is, the at least one processor may determine whether there is the broadband noise whose level is constantly maintained over a relatively wide frequency range in the frequency spectrum to have a flat shape or whether there is the broadband noise which appears in a form in which the intensity of frequency spectrum is overall increased while having a flat shape.

At step (d-3), the at least one processor may analyze a value of sub-harmonics or a value of ultra-harmonics in the frequency analysis.

In detail, the at least one processor may determine, through the frequency analysis, whether there is detected a component of the received acoustic signal generated by the motion of the microbubbles by the sub-harmonics, that is, a value corresponding to half the resonant frequency value of the ultrasound energy output from the piezoelectric element as a resonant frequency.

Alternatively, the at least one processor may determine, through the frequency analysis, whether there is detected a component of the received acoustic signal generated by the motion of the microbubbles by using the ultra-harmonics, that is, a value corresponding to n/2 times (n being a natural number being not "1") the resonant frequency value of the ultrasound energy output from the piezoelectric element as a resonant frequency.

At step (d-4), the at least one processor may determine whether the value of the sub-harmonics or the value of the ultra-harmonics reaches a preset first threshold value. When the value of the sub-harmonics or the value of the ultra-harmonics exceeds the preset first threshold value, it may be determined that there exists the phenomenon that the microbubbles near the blood-brain barrier strongly oscillate or are destroyed by ultrasound irradiation.

In this case, the first threshold value may be set in advance by the user and may have any one of various values.

At step (e), depending on whether the ultrasound energy satisfies the preset control condition, the signal control unit 14 may stop the output of the ultrasound energy or may adjust at least one of the output conditions: the duty cycle, the number of stimulus pulses per preset time, the pulse repetition frequency per preset time, and the applied voltage.

In detail, when the broadband noise is present in the frequency analysis, at step (e-1), the output of the ultrasound energy may be stopped under control of the at least one processor which drives the signal control unit 14.

When the value of the sub-harmonics or the value of the ultra-harmonics reaches the preset first threshold value, at step (e-2), the output of the ultrasound energy may be stopped under control of the at least one processor.

That is, when the frequency analysis result indicates that the broadband noise exists or when the value of the sub-harmonics or the value of the ultra-harmonics reaches the preset first threshold value, the at least one processor may determine that the excessive acoustic cavitation (or excessive bubble oscillation) occurs in the acoustic cavitation generation material.

The output of the ultrasound energy by the piezoelectric element may be stopped under control of the at least one processor, and thus, damage of perivascular tissue of the blood-brain barrier (BBB) may be minimized.

That is, because there is a risk that the acoustic cavitation generation material (or microbubbles) is destroyed, the at least one processor stops the output of the ultrasound energy.

When the broadband noise does not exist and when the value of the sub-harmonics or the value of the ultra-harmonics does not reach the preset first threshold value, at step (e-3), the at least one processor may additionally analyze a value of harmonics in the frequency analysis.

That is, through the frequency analysis, the at least one processor may detect a component of the received acoustic signal generated by the motion of the microbubbles by using the harmonics, that is, a frequency having a value corresponding to n times (n being a natural number) the resonant frequency value of the ultrasound energy output from the piezoelectric element.

At step (e-3-1), the at least one processor may determine whether the value of the harmonics reaches a preset second threshold value. In this case, the second threshold value may be set in advance by the user and may have any one of various values.

When the value of the harmonics does not reach the preset second threshold value, at step (e-3-2), the at least one processor may adjust at least one of the output conditions: the duty cycle, the number of stimulus pulses per preset time, the pulse repetition frequency per preset time, and the applied voltage.

For example, when the value of the harmonics does not reach the preset second threshold value, at step (e-3-1-1), the at least one processor may increase the applied voltage such that the intensity of the ultrasound energy to be output is increased.

That is, when the frequency analysis result indicates that the value of the harmonics does not reach the preset second threshold value, the at least one processor may determine that the excessive acoustic cavitation does not occur in the acoustic cavitation generation material (or corresponds to safe bubble oscillation or active bubble oscillation).

To open the blood-brain barrier (BBB), the at least one processor may continuously or randomly increase the applied voltage without decreasing the applied voltage, such that the intensity of the ultrasound energy to be output is increased.

Alternatively, when the value of the harmonics does not reach the preset second threshold value, the at least one processor may increase the duty cycle, the number of stimulus pulses per preset time, and the pulse repetition frequency per preset time such that the intensity of the ultrasound energy to be output is increased.

The detailed contents are the same as or similar to the increase in the applied voltage described above, and thus, additional description will be omitted to avoid redundancy.

According to the above description, the blood-brain barrier (BBB) may be safely opened.

When the value of the harmonics reaches the preset second threshold value, at step (e-3-1-2), through the frequency analysis, the at least one processor may determine whether the sub-harmonics or the ultra-harmonics exist or whether the value of the sub-harmonics or the value of the ultra-harmonics reaches a preset third threshold value.

In this case, the third threshold value may be set in advance by the user and may have any one of various values.

When the sub-harmonics or the ultra-harmonics do not exist or when the value of the sub-harmonics or the value of the ultra-harmonics does not reach the preset third threshold value, at step (e-3-1-2-1), the at least one processor may increase at least one or more of the duty cycle, the number of stimulus pulses per preset time, the preset pulse repetition frequency, and the applied voltage such that the intensity of the ultrasound energy to be output is increased.

That is, when the frequency analysis result indicates that the sub-harmonics or the ultra-harmonics do not exist or that the value of the sub-harmonics or the value of the ultra-harmonics does not reach the preset third threshold value, the at least one processor may determine that the excessive acoustic cavitation (or excessive bubble oscillation) does not occur in the acoustic cavitation generation material.

To open the blood-brain barrier (BBB), the at least one processor may increase at least one or more of the duty cycle, the number of stimulus pulses per preset time, the preset pulse repetition frequency, and the applied voltage such that the intensity of the ultrasound energy to be output is increased.

According to the above description, the blood-brain barrier (BBB) may be stably opened.

When the sub-harmonics or the ultra-harmonics exist or when the value of the sub-harmonics or the value of the ultra-harmonics reaches the preset third threshold value, at step (e-3-1-2-2), the at least one processor may determine whether a preset treatment time or a preset output time arrives.

In this case, the preset treatment time or the preset output time may be set in advance by the user and may have any one of various times.

When the preset treatment time arrives, at step (e-3-1-2-2-1), the at least one processor may stop the output of the ultrasound energy. Alternatively, when the preset output time arrives, at step (e-3-1-2-2-3), the at least one processor may stop the output of the ultrasound energy.

When the preset treatment time does not arrive, at step (e-3-1-2-2-2), the at least one processor may decrease at least one of the duty cycle, the number of stimulus pulses per preset time, the preset pulse repetition frequency, and the applied voltage such that the intensity of the ultrasound energy to be output is decreased.

Alternatively, when the preset output time does not arrive, at step (e-3-1-2-2-4), the at least one processor may decrease at least one of the duty cycle, the number of stimulus pulses per preset time, the preset pulse repetition frequency, and the applied voltage such that the intensity of the ultrasound energy to be output is decreased.

That is, when the preset treatment time does not arrive or when the preset output time does not arrive, for the safe opening of the blood-brain barrier (BBB), the at least one processor may decrease the intensity of the ultrasound energy to be output.

Referring to FIG. 2B, an example in which each of the first to third threshold values has a preset value, the magnitude (or intensity) of the third threshold value is smaller than the magnitude (or intensity) of the first threshold value, and the magnitude (or intensity) of the second threshold value is greater than the magnitude (or intensity) of the third threshold value is illustrated. However, the present disclosure is not limited thereto.

That is, as described above, the magnitudes of the first to third threshold values may be set by the setting of the user to be different from or identical to each other. The intensity of the ultrasound energy to be output may be adjusted depending on the closed loop feedback of FIG. 2A based on a result of comparing and analyzing the magnitudes of the first to third threshold values with the value of the sub-harmonics, the value of the ultra-harmonics, or the value of the harmonics.

As described above, to open the blood-brain barrier (BBB) the therapeutic ultrasonication system 1 for blood-brain barrier opening according to an embodiment of the present disclosure may set the output condition of the ultrasound energy and may output the corresponding ultrasound energy to the acoustic cavitation generation material near the blood-brain barrier.

Also, the therapeutic ultrasonication system 1 may receive (or collect) the acoustic signal due to the acoustic cavitation phenomenon of the acoustic cavitation generation material and may compare the acoustic signal with the control condition for adjusting the ultrasound energy.

That is, the therapeutic ultrasonication system 1 may adjust at least one or more of the duty cycle of the ultrasound energy, the number of stimulus pulses per preset time, the preset pulse repetition frequency, or the applied voltage to be supplied to the piezoelectric element based on the comparison result with the control condition.

According to the above description, it may be possible to stably open the blood-brain barrier while minimizing damage of perivascular tissue of the blood-brain barrier.

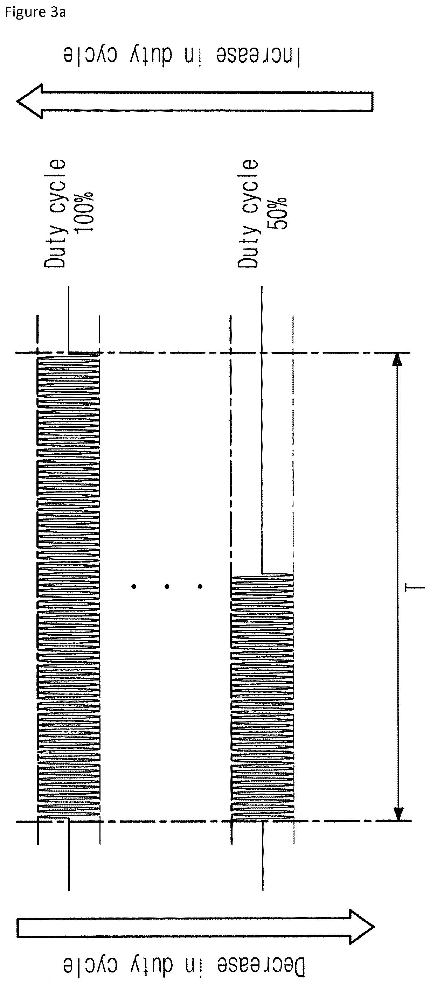

FIG. 3A is a diagram illustrating ultrasound energy according to a change in a duty cycle, according to an embodiment of the present disclosure.

The duty cycle means a ratio of a total time during which the pulse continues to a specific time T in which a continuous operation of the pulse is performed.

For example, in FIG. 3A, when the duty cycle is 100%, the oscillation of the ultrasound energy pulse continues during the specific time T. Meanwhile, when the duty cycle is 50%, the oscillation of the ultrasound energy pulse continues only during T/2 corresponding to half the specific time T.

As the duty cycle increases, during the specific time T, the ratio of the total duration of the pulse oscillation may increase, and the intensity of ultrasound energy may increase. As the duty cycle decreases, during the specific time T, the ratio of the total duration of the pulse oscillation may decrease, and the intensity decrease ultrasound energy may decrease.

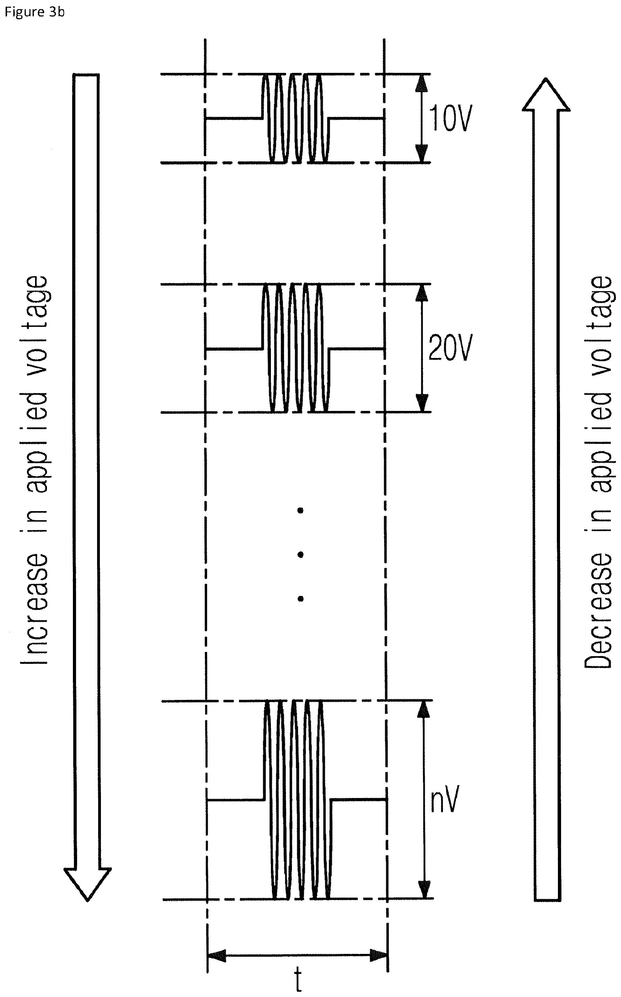

FIG. 3B is a diagram illustrating ultrasound energy according to a change in an applied voltage, according to an embodiment of the present disclosure.

In FIG. 3B, as the applied voltage increases (e.g., to 10V, 20V, ..., nV (n being an integer)), the magnitude of the amplitude of the ultrasound energy output during a specific time t may increase, and the intensity of the ultrasound energy may increase. As the applied voltage decreases (e.g., to nV, ..., 20V, and 10V), the magnitude of the amplitude of the ultrasound energy output during the specific time t may decrease, and the intensity of the ultrasound energy may decrease.

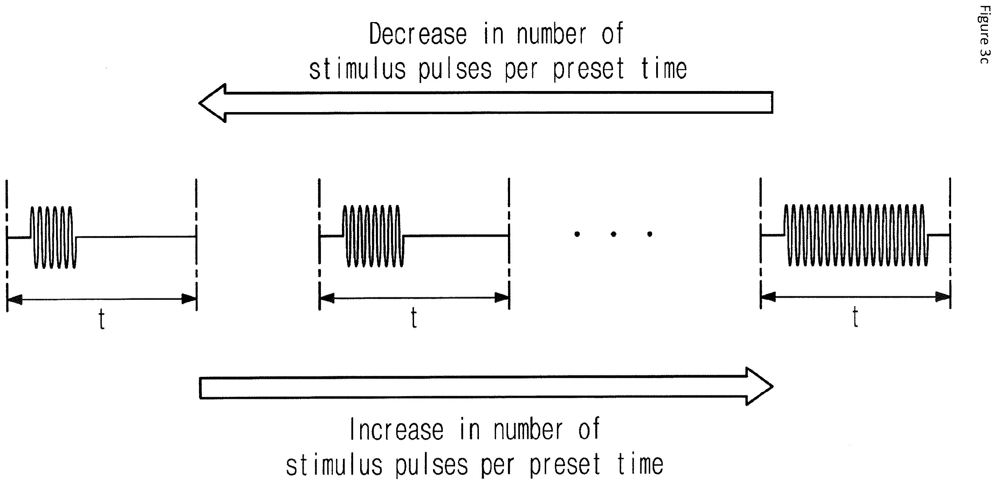

FIG. 3C is a diagram illustrating ultrasound energy according to a change in the number of stimulus pulses per preset time, according to an embodiment of the present disclosure.

The number of stimulus pulses per preset time means the number of pulses of the ultrasound energy oscillating during the specific time t.

As the number of stimulus pulses per preset time increases, the number of pulses of the ultrasound energy oscillating during the specific time t may increase, and the intensity of the ultrasound energy may increase. As the number of stimulus pulses per preset time decreases, the number of pulses of the ultrasound energy oscillating during the specific time t may decrease, and the intensity of the ultrasound energy may decrease.

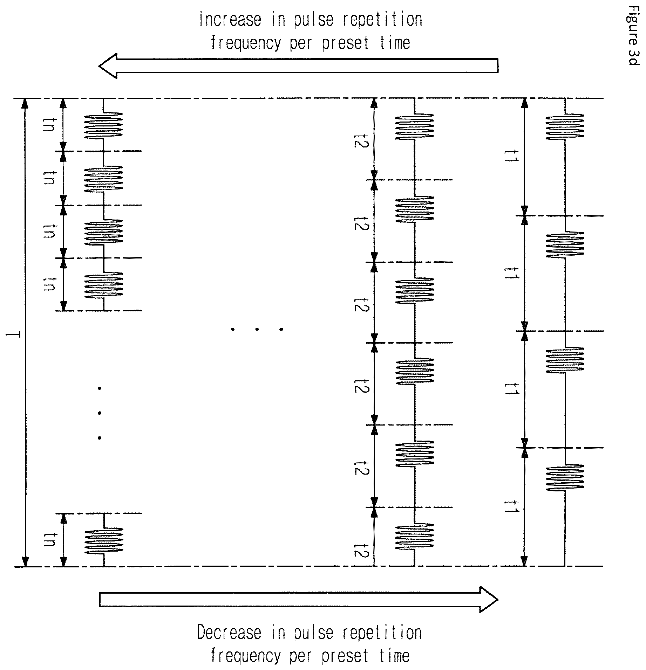

FIG. 3D is a diagram illustrating ultrasound energy according to a change in a pulse repetition frequency per preset time, according to an embodiment of the present disclosure.

The pulse repetition frequency per preset time means the frequency of the ultrasound energy repeated during the specific time T.

As the pulse repetition frequency per preset time increases, the frequency of the ultrasound energy repeated during the specific time T increases. As the pulse repetition frequency per preset time decreases, the frequency of the ultrasound energy repeated during the specific time T decreases.

That is, as the pulse repetition frequency per preset time increases, the period (t1, t2, ..., tn) repeated during the specific time T decreases. Because the oscillation period of the ultrasound energy becomes shorter based on the specific time T, the number of repeated oscillations of the ultrasound energy increases.

In contrast, as the pulse repetition frequency per preset time decreases, the period (tn, ..., t2, and t1) repeated during the specific time T increase. Because the oscillation period of the ultrasound energy becomes longer based on the specific time T, the number of repeated oscillations of the ultrasound energy decreases.

As described above, according to an embodiment of the present disclosure, the at least one processor which drives the signal generation condition setting unit 10 may be provided with a signal (or control signal), which indicates whether the control condition is satisfied, from the at least one processor, which drives the signal control unit 14, in real time during the output of the ultrasound energy.

The signal generation condition setting unit 10 may generate a signal for adjusting the above output conditions (the duty cycle, the number of stimulus pulses per preset time, the pulse repetition frequency per preset time, and the applied voltage) described with reference to FIGS. 3A to 3D based on the control signal and may provide the generated signal to the signal generation unit 11.

The signal generation unit 11 may control the piezoelectric element based on the received signal such that the ultrasound energy having a relevant output condition is output; in this case, under control of the signal generation unit 11, damage of perivascular tissue of the blood-brain barrier (BBB) is minimized, and the effect of blood-brain barrier opening is maximized.

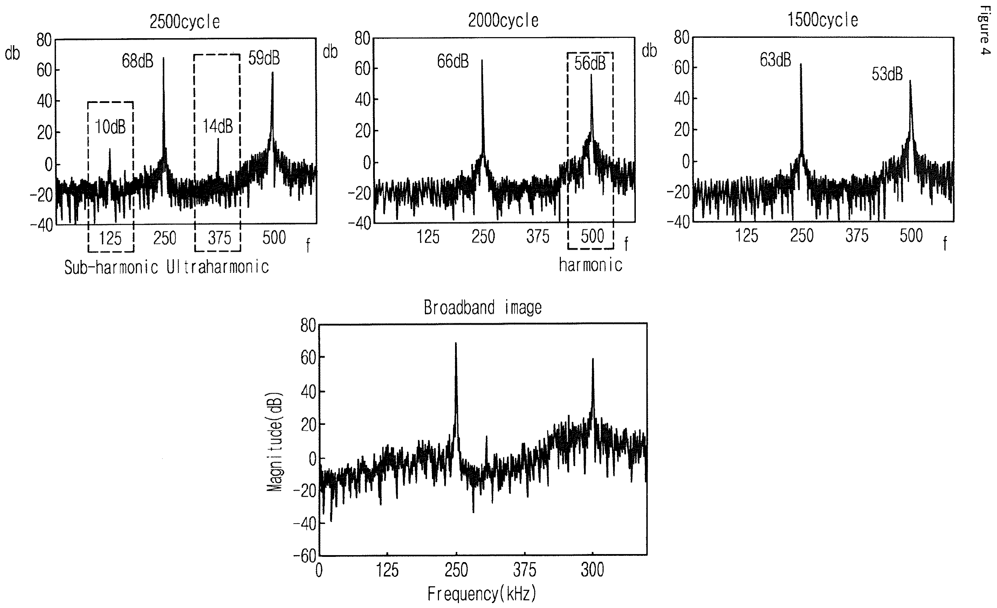

FIG. 4 is a diagram illustrating an analysis result of an acoustic signal according to a change in the number of stimulus pulses, according to an embodiment of the present disclosure.

The case of adjusting the duty cycle or the pulse repetition frequency per time among the output conditions in association with the ultrasound energy to be output is the same as or similar to the case of adjusting the number of stimulus pulses (or the number of stimulus pulses per time) to be described below in association with the ultrasound energy to be output, and thus, additional description will be omitted to avoid redundancy.

FIG. 4 shows a result of receiving and analyzing acoustic signal due to the acoustic cavitation phenomenon of the acoustic cavitation generation material when an ultrasound energy pulse with the specific number of stimulus pulses (1500 cycles, 2000 cycles, or 2500 cycles) is applied during a preset time.

When pulses, the number of which is 1500 cycles, are output to the acoustic cavitation generation material during the preset time, it is confirmed from the analyzed result that a harmonic component (a frequency of 500 KHz) of the resonant frequency (250 KHz) of the ultrasound energy output is detected.

Meanwhile, it is confirmed that as the number of pulses increases (in FIG. 4, 2000 cycles, 2500 cycles, ...), the amplitude of an electrical signal corresponding to the harmonic component increases (53db, 56db, 59db, ...).

That is, when the electrical signal corresponding to the harmonic component does not reach a preset specific threshold value, the acoustic cavitation generation material near the blood-brain barrier shows the stable oscillation phenomenon due to the ultrasound energy, and the at least one processor may determine that the blood-brain barrier is in an open state.

In this case, as described with reference to FIGS. 1 and 2, the at least one processor may increase the intensity of the ultrasound energy to be output by gradually or temporarily increasing the applied voltage to be supplied to the piezoelectric element, increasing the duty cycle, or decreasing the pulse repetition frequency. According to the above description, the blood-brain barrier may be stably opened.

Meanwhile, when pulses, the number of which is 1500 cycles, are output to the acoustic cavitation generation material during the preset time, it is confirmed from the analyzed result that a sub-harmonic component (a frequency of 125 KHz) or an ultra-harmonic component (a frequency of 375 KHz) of the resonant frequency (250 KHz) of the ultrasound energy output is not detected.

As the number of pulses increases (in FIG. 4, 2000 cycles, 2500 cycles, ...) (or the number of stimulus pulses per preset time increases), the amplitude of an electrical signal corresponding to the sub-harmonic component or the ultra-harmonic component increases (0db → 10db, 0db → 14db)

In this case, when the electrical signal corresponding to the sub-harmonic component or the ultra-harmonic component does not reach the preset specific threshold value, the acoustic cavitation generation material near the blood-brain barrier shows the active oscillation phenomenon due to the ultrasound energy, and it may be determined that the blood-brain barrier is in an open state.

As described with reference to FIGS. 1 and 2, the at least one processor may increase the intensity of the ultrasound energy by adjusting (or increasing) at least one or more of the output conditions of the ultrasound energy output from the piezoelectric element, that is, at least one or more of the duty cycle, the number of stimulus pulses per time, the pulse repetition frequency per time, and the applied voltage. According to the above description, the opening of the blood-brain barrier may be stably performed.

However, when the magnitude of the electrical signal corresponding to the sub-harmonic component or the ultra-harmonic component reaches the preset specific threshold value, the acoustic cavitation generation material near the blood-brain barrier shows the excessive oscillation phenomenon due to the ultrasound energy and thus may cause damage of perivascular tissue of the blood-brain barrier.

As described with reference to FIGS. 1 and 2, the at least one processor may decrease the intensity of the ultrasound energy by adjusting (or decreasing) at least one or more of the output conditions of the ultrasound energy output from the piezoelectric element, that is, at least one or more of the duty cycle, the number of stimulus pulses per time, the pulse repetition frequency per time, and the applied voltage. According to the above description, the opening of the blood-brain barrier may be stably performed without damaging the perivascular tissue of the blood-brain barrier.

Meanwhile, it may be confirmed from the analyzed result that, when the broadband noise is detected, the magnitude appears to overall increase in a flat shape while maintaining a constant level in a relatively wide frequency range, compared to "1500cycle", "2000cycle", and "2500cycle".

This means that the phenomenon that the microbubbles near the blood-brain barrier are destroyed appears. That is, there is a risk that the perivascular tissue of the blood-brain barrier is damaged.

In this case, as described with reference to FIGS. 1 and 2, the output of the ultrasound energy may be stopped under control of the at least one processor.

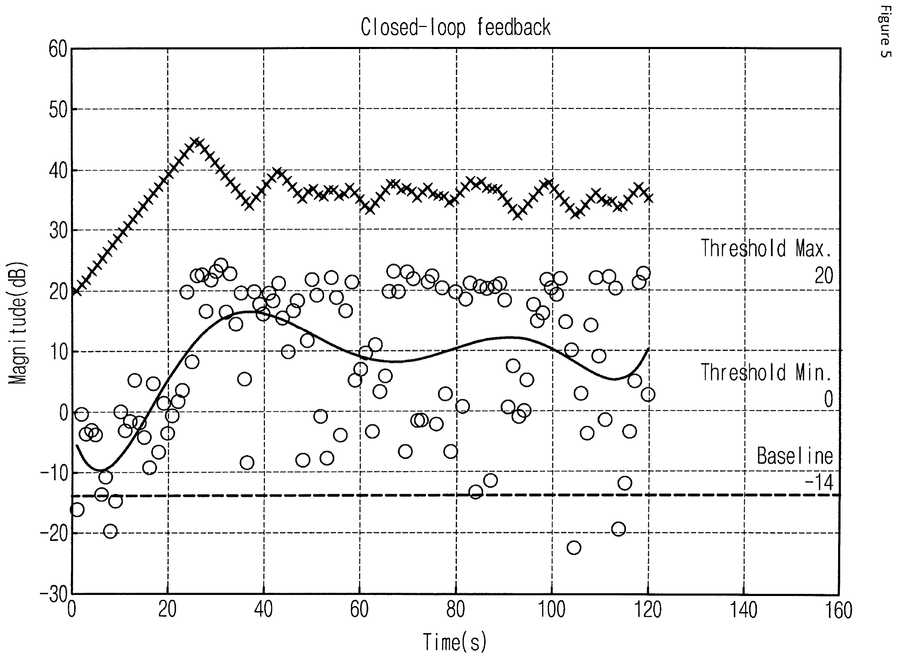

FIG. 5 is a diagram illustrating an analysis result of an acoustic signal according to closed-loop feedback, according to an embodiment of the present disclosure.

Referring to FIG. 5, a graph line illustrated on top indicates a trend of an applied voltage supplied to the piezoelectric element. A solid line below the top graph line indicates a result of analyzing an acoustic signal generated by the acoustic cavitation phenomenon of the acoustic cavitation generation material.

It may be confirmed that the trend of the graph indicating the analysis of the acoustic signal over time follows the trend of the graph of the applied voltage. That is, as the applied voltage supplied to the piezoelectric element increases, the intensity of the received acoustic signal increases; as the applied voltage decreases, the intensity of the received acoustic signal decreases.

Meanwhile, when a given time (20s) elapses after the applied voltage is supplied, it may be confirmed that the graph indicating the analysis of the acoustic signal is only between a threshold Max value and a threshold Min value.

This means that the intensity of ultrasound energy is controlled by the ultrasonic wave control method (or closed loop feedback) of the therapeutic ultrasonication system for blood-brain barrier opening according to the present disclosure within a range (the threshold Max value to the threshold Min value) in which the microbubbles are capable of stably opening the blood-brain barrier without being destroyed.

That is, the therapeutic ultrasonication system for blood-brain barrier opening according to the present disclosure and the control method of the therapeutic ultrasonication system may control the output of the ultrasound energy based on the acoustic signal received from the microbubble near the blood-brain barrier, and thus, it may be possible to minimize damage of the perivascular tissue of the blood-brain barrier and also to open the blood-brain barrier stably.

The drawings and the detailed description of the present disclosure given so far are merely illustrative of the present disclosure. This is only used for the purpose of describing the present disclosure and is not used to limit the meaning or the scope of the present disclosure described in claims. Therefore, it will be understood that various modifications and other equivalent embodiments are possible from this point by one skilled in the art. The technical protection scope of the present disclosure will be defined by the technical spirit of the appended claims.

The above embodiments may be implemented with hardware components, software components, and/or a combination of hardware components and software components. For example, the devices, methods, components, and elements described in the embodiments may be implemented by using one or more general-purpose computers or special-purpose computers, such as a processor, a controller, an arithmetic logic unit (ALU), a digital signal processor, a microcomputer, a field programmable array (FPA), a programmable logic unit (PLU), a microprocessor, or any device which may execute an instruction and respond thereto.

A processing unit may execute an operating system (OS) or one or software applications running on the OS. Also, the processing unit may access, store, manipulate, process, and generate data in response to the execution of software. For convenience of understanding, the description is given as a single processing unit is used, but it will be understood by one skilled in the art that the processing unit may include a plurality of processing elements and/or a plurality of types of processing elements.

For example, the processing unit may include a plurality of processors or may include one processor and one controller. Also, any other processing configuration such as a parallel processor is possible. Software may include a computer program, a code, an instruction, or one or more combinations thereof, and may implement a processing device to operate in a desired manner or may individually or collectively instruct the processing device.

Software and/or data may be embodied in any type of a machine, a component, physical equipment, virtual equipment, a computer storage medium, or a device so as to be interpreted by the processing unit or to provide instructions or data to the processing unit. Software may be distributed throughout computer systems connected via a network so as to be stored or executed in a distribution manner. Software and data may be recorded in one or more computer-readable storage media.

The methods according to the embodiments may be recorded in a computer-readable medium including program instructions executable through various computer devices. A computer-readable medium may include a program instruction, a data file, a data structure, etc. independently or may include a combination thereof. The program instruction recorded in the medium may be designed and configured specially for the embodiments or may be known and available to one skilled in computer software.

The computer-readable medium may include, for example, a hardware device, which is specially configured to store and execute a program instruction, such as magnetic media (e.g., a hard disk drive, a floppy disk, and a magnetic tape), optical media (e.g., CD-ROM and DVD), a read only memory (ROM), a random access memory (RAM), and a flash memory. As an example, the program instruction includes not only a machine language code created by a compiler but also a high-level language code capable of being executed by a computer by using an interpreter or the like. The above hardware device may be configured to act as one or more software modules to perform the operations of the embodiment, and vice versa.

Even though the above embodiments are described with reference to some drawings and embodiments, it will be apparent to one skilled in the art that various modifications and variations may be made from the foregoing description. For example, the described technologies are performed in an order different from that of the described method, and/or appropriate results may be obtained even though the described components such as a system, a structure, a device, and a circuit are united or combined in a form different from that of the described method or are substituted or replaced by any other components or equivalents. Therefore, other implementations, other embodiments, and equivalents to claims are within the scope of the following claims.

## Claims

1. An ultrasonic wave control method of a therapeutic ultrasonication system for blood-brain barrier opening, which is driven by at least one processor, the method comprising the steps of:
(a) setting, by the at least one processor, an output condition for outputting ultrasound energy to an acoustic cavitation generation material injected into an object;
(b) outputting, by the at least one processor, ultrasound energy corresponding to the output condition to the object;
(c) receiving, by the at least one processor, an acoustic signal due to an acoustic cavitation phenomenon of the acoustic cavitation generation material;
(d) analyzing, by the at least one processor, the acoustic signal to determine whether a preset control condition is satisfied; and
(e) depending on whether the preset control condition is satisfied, stopping, by the at least one processor, the output of the ultrasound energy or adjusting at least one of output conditions to output the ultrasound energy, wherein the output conditions include a duty cycle, the number of stimulus pulses per preset time, a pulse repetition frequency per preset time, and an applied voltage.

2. The method of claim 1, wherein the (a) step includes steps of:
(a-1) setting an initial center frequency or an initial applied voltage among the output conditions; and
(a-2), setting at least one of an initial duty cycle, the initial number of stimulus pulses per preset time, and an initial pulse repetition frequency per preset time among the output conditions.

3. The method of claim 2, wherein the (b) step includes:
outputting, to the object, the ultrasound energy having at least one of the set initial center frequency, the set initial duty cycle, the set initial number of stimulus pulses, and the set initial pulse repetition frequency or the ultrasound energy generated by applying the set initial applied voltage, and
wherein the (c) step includes:
receiving the acoustic signal from the acoustic cavitation generation material injected into the object.

4. The method of claim 1, wherein the (d) step includes steps of:
(d-1) performing a frequency analysis on the acoustic signal;
(d-2) determining whether a broadband noise is present in the frequency analysis;
(d-3) analyzing a value of sub-harmonics or ultra-harmonics in the frequency analysis; and
(d-4) analyzing the value of the sub-harmonics or the ultra-harmonics to determine whether to reach a preset first threshold value.

5. The method of claim 4, wherein the (e) step includes steps of:
(e-1) stopping the output of the ultrasound energy, when the broadband noise exists;
(e-2) stopping the output of the ultrasound energy, when the value of the sub-harmonics or the ultra-harmonics reaches the preset first threshold value; and
(e-3) analyzing a value of harmonics in the frequency analysis, when the broadband noise does not exist and when the value of the sub-harmonics or the ultra-harmonics does not reach the preset first threshold value.

6. The method of claim 5, wherein the (e -3) step includes steps of:
(e-3-1) determining whether the value of the harmonics reaches a preset second threshold value; and
(e-3-2) adjusting at least one of the duty cycle, the number of stimulus pulses per preset time, the pulse repetition frequency per preset time, and the applied voltage, when the value of the harmonics does not reach the preset second threshold value.

7. The method of claim 6, wherein the (e-3-1) step includes steps of:
(e -3 -3-1) increasing at least one of the duty cycle, the number of stimulus pulses per preset time, the pulse repetition frequency per preset time, and the applied voltage such that an intensity of the ultrasound energy to be output is increased, when the value of the harmonics does not reach the preset second threshold value; and
(e-3-1-2) determining whether the sub-harmonics or the ultra-harmonics exist or whether the value of the sub-harmonics or the value of the ultra-harmonics reaches a preset third threshold value, when the value of the harmonics reaches the preset second threshold value.

8. The method of claim 7, wherein the (e-3-1-2) step includes steps of:
(e-3-1-2-1) increasing at least one or more of the duty cycle, the number of stimulus pulses per preset time, the pulse repetition frequency per preset time, and the applied voltage such that the intensity of the ultrasound energy to be output is increased, when the sub-harmonics or the ultra-harmonics do not exist or when the value of the sub-harmonics or the value of the ultra-harmonics does not reach the preset third threshold value; and
(e-3-1-2-2) determining whether a preset treatment time or a preset output time arrives, when the sub-harmonics or the ultra-harmonics exist or when the value of the sub-harmonics or the value of the ultra-harmonics reaches the preset third threshold value.

9. The method of claim 8, wherein the (e-3-1-2-2) step includes steps of:
(e-3-1-2-2-1) stopping the output of the ultrasound energy, when the preset treatment time arrives; and
(e-3-1-2-2-2) decreasing at least or more of the duty cycle, the number of stimulus pulses per preset time, the pulse repetition frequency per preset time, and the applied voltage such that the intensity of the ultrasound energy to be output is decreased, when the preset treatment time does not arrive.

10. The method of claim 8, wherein the (e-3-1-2-2) step includes steps of:
(e-3-1-2-2-3) stopping the output of the ultrasound energy, when the preset output time arrives; and
(e-3-1-2-2-4), decreasing at least one of the duty cycle, the number of stimulus pulses per preset time, the preset pulse repetition frequency, and the applied voltage such that the intensity of the ultrasound energy to be output is decreased, when the preset output time does not arrive.

11. A non-transitory computer-readable recording medium storing a program for executing the ultrasonic wave control method of the therapeutic ultrasonication system for blood-brain barrier opening of claim 1.

12. A therapeutic ultrasonication system for blood-brain barrier opening, comprising:
a signal generation condition setting unit configured to set an output condition for outputting ultrasound energy to an acoustic cavitation generation material injected into an object;
a signal generation unit, wherein the ultrasound energy corresponding to the output condition is output to the object under control of the signal generation unit;
a signal collection unit configured to receive an acoustic signal due to an acoustic cavitation phenomenon of the acoustic cavitation generation material;
a frequency analysis unit configured to analyze the acoustic signal to determine whether to satisfy a preset control condition; and
a signal control unit configured to stop the output of the ultrasound energy or to adjust at least one of output conditions of the ultrasound energy, depending on whether the preset control condition is satisfied, wherein the output conditions include a duty cycle, the number of stimulus pulses per preset time, a pulse repetition frequency per preset time, and an applied voltage.
